(19) Europäisches Patentamt
European Patent Office
Office européen des brevets

(11) Numéro de publication: **0 013 196**
**B1**

# (12) FASCICULE DE BREVET EUROPEEN

(45) Date de publication du fascicule de brevet: **25.11.81**

(51) Int. Cl.$^3$: **C 07 C 51/60** //B01J31/02

(21) Numéro de dépôt: **79400878.9**

(22) Date de dépôt: **15.11.79**

## (54) Procédé de fabrication des chlorures d'acides organiques.

(30) Priorité: **29.11.78 FR 7833670**
**30.08.79 FR 7921730**

(43) Date de publication de la demande:
**09.07.80 Bulletin 80/14**

(45) Mention de la délivrance du brevet:
**25.11.81 Bulletin 81/47**

(84) Etats Contractants Désignés:
**DE FR GB IT**

(56) Documents cités:
**FR - A - 2 196 305**
**US - A - 3 544 626**
**US - A - 3 810 940**

(73) Titulaire: **SOCIETE NATIONALE DES POUDRES ET EXPLOSIFS**
**12, quai Henri IV**
**F-75181 PARIS CEDEX 04 (FR)**

(72) Inventeur: **Lecolier, Serge**
**3, allée des Cartelines**
**F-91510 Janville sur Juine (FR)**
Inventeur: **Senet, Jean-Pierre**
**79, rue de la Gare Herbeauvilliers-Buthiers**
**F-77760 La Chapelle la Reine (FR)**

**EP 0 013 196 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de fabrication des chlorures d'acides organiques

La présente invention se rapporte à un procédé de fabrication des chlorures d'acides organiques par phosgénation des acides organiques ou des anhydrides d'acides organiques correspondants, en présence d'un catalyseur.

Il est connu d'utiliser un catalyseur pour rendre possible la production de chlorures d'acides par action du phosgène sur les acides ou anhydrides correspondants, à une température raisonnable et à une pression voisine de la pression atmosphérique.

Dans les brevets français 732 078 et 2 212 319 on a ainsi proposé d'utiliser des amines tertiaires. Toutefois, ces derniers catalyseurs ayant une activité très moyenne, nécessitant par conséquent l'emploi d'une température élevée et donnant lieu dans certains cas à la formation de sous produits gênants, d'autres solutions furent proposées

Ainsi, on a proposé d'employer des amides, des urées, des thiourées ou des pyrrolidones, tels que le diméthylformamide, la tétraméthylurée, la tétraméthylthiourée, les N-alkylpyrrolidones, comme décrit notamment dans le brevet allemand 1 026 750 les brevets américains 3 318 950 et 3 544 627 et le brevet français 2 196 305. Ces substances, si elles apportent un large progrès du point de vue de l'activité catalytique ont l'inconvénient de conduire, après quelques recyclages, à la formation de boues qui obturent les canalisations et généralement aussi à la formation de sous-produits secondaires parfois toxiques et souvent difficiles à séparer du chlorure d'acide.

Parallèlement, les catalyseurs minéraux, comme le charbon actif, et les bases de Lewis, ont l'avantage de donner peu ou pas de sous-produits, mais possèdent en revanche une activité catalytique réduite imposant l'emploi de températures de réaction élevées. De tels catalyseurs sont décrits dans les brevets américains 2 156 177 et 2 272 299 et ne constituent pas une solution satisfaisante du fait des difficultés technologies (filtration, distillation) impliquées par la présence de solides dans le réacteur.

Deux solutions presque satisfaisantes ont été plus récemment proposées dans les brevets américains 3 547 960 et 3 869 485, d'une part, et le brevet américain 3 962 326, d'autre part. Selon le brevet américain 3 547 960, on utilise un membre de la famille des imidazoles. On obtient alors une réaction rapide sans formation de sous-produits. Malheuresement, dans certains cas, on constate qu'il se forme deux phases dans le réacteur, ce qui devient gênant au bout de quelques recyclages. Selon le brevet américain 3 962 326, on emploie un oxyde ou un sulfure de phosphine tertiaire. Dans ce cas, l'action catalytique est bonne même avec de faibles taux de catalyseurs et il se forme peu ou pas de sous-produits dans la mesure où le catalyseur n'est pas recyclé. En effet, dans ce dernier cas, le dichlorure de phosphine tertiaire qui est responsable de la chloruration et qui se forme par action du phosgène sur l'oxyde de phosphine, d'après DE 1 192 215, se décompose partiellement au moment de la distillation du chlorure d'acide formé. Il s'ensuit que la charge de catalyseur ne peut être recyclé de manière satisfaisante.

On voit donc qu'il reste encore à découvrir des catalyseurs ayant à la fois une bonne activité à une température raisonnable, et une bonne aptitude aux recyclages et ne conduisant pas à la formation de sous-produits, dans une proportion gênante.

La Demanderesse a maintenant découvert une famille de catalyseurs répondant aux qualités précédemment citées et dont l'intérêt industriel est par conséquent trés grand.

L'invention concerne un procédé de fabrication de chlorures d'acides organiques par phosgénation à chaud des acides ou des anhydrides d'acides correspondants en présence d'un catalyseur, caractérisé en ce qu'on utilise comme catalyseur au moins un membre de la famille de formule générale (I)

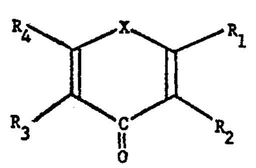

où X est un atome d'oxygène ou un atome d'azote monosubstitué, par un groupe $R_5$ qui est un groupe alkyle comprenant de 1 à 6 atomes de carbone, et où $R_1$, $R_2$, $R_3$, $R_4$ sont tous différents ou sont semblables pour au moins deux d'entre eux et sont un atome d'hydrogène ou un radical organique susceptible ou non de réagir avec le phosgène. De préférence quand les radicaux $R_1$, $R_2$, $R_3$ et/ou $R_4$ sont des radicaux organiques, ils ne contiennent pas plus de 20 atomes de carbone.

On désigne dans tout de qui suit par catalyseurs les membres de la famille de formule générale (I)

Parmi les catalyseurs concernés par le procédé selon la présente invention, certains sont préférés pour leur activité catalytique élevée. Ainsi, on préfère notamment les catalyseurs de formule générale (I) dans laquelle d'une part $X = O$ ou $N—R_5$, $R_5$ étant un groupe alkyl linéaire ou ramifié de $C_1$ à $C_6$, et d'autre part

$R_1 = H$, alkyl linéaire ou ramifié de $C_1$ à $C_{12}$, substitué ou non par des atomes de chlore, des hydroxyles, des carboxyles, des groupes esters ou des groupes éthers, aryle substitué ou non par un ou des groupes halogène, hydroxyle ou carboxyle, aralkyle de $C_7$ à $C_{20}$, alkaryle de $C_7$ à $C_{20}$,

hydroxyle, carboxyle, dialkylamino de $C_1$ à $C_3$,

$R_2$ et $R_3$ sont identiques ou différents et ont l'une des significations possibles pour $R_1$ et, éventuellement, la même signification que $R_1$, ou lorsque $X = N—R_5$ sont de préférence l'un et l'autre un atome d'hydrogène,

$R_4$ a l'une des significations possibles pour $R_1$ et éventuellement a la même signification que $R_1$, et/ou que $R_2$ et/ou $R_3$,

ou bien dans laquelle $R_1$ et $R_2$ pris ensemble et/ou $R_3$ et $R_4$ pris ensemble forment un groupe phényle.

Selon un mode de réalisation particuliérement préferé de l'invention, on utilise comme catalyseur des composés de formule générale (I) dans laquelle d'une part $X = O$ ou $N—R_5$, $R_5$ étant un groupe alkyl linéaire ou ramifié de $C_1$ à $C_6$ et d'autre part:

$R_1 = R_2 = R_3 = R_4 = H$

ou bien dans laquelle:

$R_1 = R_4 =$ alkyl de $C_1$ à $C_6$, et $R_2 = R_3 = H$

ou bien dans laquelle $X = O$ et:

$R_1 = R_4 = H$ et $R_2$, $R_3 =$ alkyle de $C_1$ à $C_6$

$R_1 = R_4 = H$ et $R_2$, $R_3 =$ aryle substitué ou non par des groupes halogène, hydroxyle ou carboxyle,

$R_1 = R_4 = H$ et $R_2$, $R_3 =$ aralkyle de $C_7$ à $C_{20}$ ou alkaryle de $C_7$ à $C_{20}$

$R_1 = R_4 = COOH$ et $R_2$, $R_3 = H$ ou alkyle de $C_1$ à $C_6$.

D'une manière générale, doivent être considérés comme entrant dans le cadre de l'invention, tous composés substitués par des groupes non réactifs vis à vis du phosgène ou par des groupes réactifs vis à vis du phosgène mais se transformant, après action du phosgène en une molécule stable ayant conservé le squelette principal:

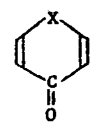

D'une manière surprenante, il a été constaté que si tous les composés pré-cités ont une activité comme catalyseur de la phosgénation des acides, certains d'entre eux ont une activité beaucoup plus marquée que d'autres du point de vue de l'accélération de la vitesse de réaction.

Ainsi, d'une manière générale, il a été trouvé que les catalyseurs selon l'invention sont particulièrement énergiques tant vis à vis de la réaction du phosgène sur l'acide que vis à vis de la réaction du phosgène sur l'anhydride correspondant, que ce dernier soit le réactif de départ ou bien formé in situ en cours de phosgénation, par action du chlorure d'acide déjà formé sur l'acide de départ. Par ailleurs, il a été trouvé qu'un catalyseur selon l'invention est d'autant plus actif, d'une manière générale, qu'il comporte des substituants $R_2$ et $R_3$ peu encombrants tels que H, la nature des substituants $R_1$ et $R_4$ ayant quant à elle une moindre importance, quoique les groupes $R_1$ et $R_4$ doivent, de préférence, être également peu encombrants.

Comme il a été dit, on peut parfaitement utiliser des catalyseurs selon l'invention réactifs vis à vis du phosgène du fait des substituants qu'ils portent. Toutefois, leur usage n'est pas préféré car il entraine une consommation supplémentaire inutile de phosgène et l'obtention d'une molécule qui, bien qu'efficace, a une efficacité en moyenne moindre que celle des catalyseurs de départ.

Les acides carboxyliques auxquels s'applique le présent procédé sont notamment les acides carboxyliques aliphatiques de 2 à 20 atomes de carbone et les acides aromatiques ou cycloaliphatiques comprenant de 4 à 24 atomes de carbone. Les acides précédents concernés sont ceux comprenant de 1 à 3 groupes carboxyle. Les anhydrides des acides précédents sont également concernés par la présente invention. A titre d'exemple, on peut citer les acides typiques suivants: acide acétique, acide butyrique, acide éthyl-2 hexanoïque, acide 3,5,5-triméthylhexanoïque, acide laurique, acide palmitique, acide stéarique, acide cyclohexane, acide 1,4-cyclohexane-dicarboxylique, acide benzoïque, acides chlorobenzoïques, acides nitrobenzoïques, acides chloro-nitro-benzoïques, acide phtalique, acide téréphtalique, acide isophtalique, acide trimellique, acide cyclohexane dicarboxylique, acides carboxyliques $\alpha,\beta$-insaturés. La phosgénation d'acides carboxyliques $\alpha,\beta$ insaturés tels que l'acide acrylique donne des chlorures d'acides carboxyliques $\alpha,\beta$-insaturés, ou, à la suite d'une addition de chlorure d'hydrogène, des chlorures d'acides $\beta$-chlorocarboxyliques.

Selon la présente invention des quantités de catalyseur de 0,01 à 10% de préférence de 0,1 à 2% en poids par rapport à l'acide carboxylique ou son anhydride sont utilisées pour obtenir l'achèvement de la réaction en une période de temps avantageusement courte. Il a été observé que la vitesse globale de la réaction augmente avec la proportion de catalyseur utilisée dans le milieu.

La gamme des températures utilisées pour la préparation des chlorures d'acides selon le procédé selon l'invention dépend pour une large part de l'acide ou de l'anhydride de départ. On utilise avantageusement une température comprise entre 70 et 180°C, et de préférence entre 90°C et 150°C. En règle générale, on préfère utiliser une température supérieure au point de fusion de l'acide ou de l'anhydride de départ. Toutefois, on peut tout aussi bien effectuer la réaction sur des acides ou des anhydrides en suspension ou en solution dans un solvant ou un agent de suspension organique inerte vis à vis du phosgène, tel qu'un solvant aromatique. On peut également utiliser comme solvant

un chlorure d'acide, en particulier, le même chlorure d'acide qu'on cherche à préparer, issu d'une fabrication précédente.

Le procédé selon l'invention fonctionne aussi bien en discontinu qu'en continu et n'implique pas, pour être appliqué, de modifications aux appareillages connus par l'homme de l'art. Le procédé peut fonctionner en utilisant une pression supérieure à la pression atmosphérique mais les catalyseurs selon l'invention ont une qualité telle qu'il n'est en général pas nécessaire de recourir à cet expédient.

La durée de la réaction est variable selon la nature de l'acide ou de l'anhydride traité et selon le degré d'avancement et la pureté que l'on souhaite atteindre. Selon le présent procédé une durée de 2 à 10 heures est suffisante pour obtenir des chlorures d'acides ayant la pureté habituelle requise pour leurs applications, avec un excellent rendement.

Quant aux proportions relatives de réactifs à employer conformément au présent procédé, elles ne diffèrent pas des procédés classiques, à savoir qu'on utilise un rapport molaire phosgène/groupe acide ou anhydride présent dans la molécule, environ égal à 1; un léger excès de phosgène étant souhaitable pour compenser les pertes par entrainement gazeux (HCl, $CO_2$).

On voit donc que les catalyseurs utilisés dans le cadre du procédé selon l'invention ont une activité catalytique comparables à celles des meilleurs catalyseurs connus. Toutefois, les catalyseurs selon l'invention ont en plus cet avantage très important industriellement qu'ils possèdent une excellente stabilité thermique autorisant plusieurs recyclages sans perte notable d'activité catalytique et permettant également de séparer et de purifier, sans que soit nécessaire un contrôle attentif de la température, les chlorures d'acides par distillation du brut réactionnel. Il résulte donc de l'emploi de ces catalyseurs un abaissement des coûts de fabrication et une amélioration de la productivité.

Les catalyseurs selon la présente invention sont des composés bien connus dont la synthèse a eté décrite dans la littérature ouverte. On peut citer, pour les composés où $X = N—R_5$ par exemple, la synthèse rapportée dans l'article de A.F. EL KASCHEF et M.H. NOSSEIR dans le Journal of American Chemical Society (1960), 82, pages 4344—47, consistant à faire agir un amine primaire de formule $R_5NH_2$ sur une $\gamma$-pyrone de formule

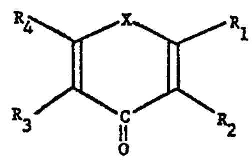

Celles-ci peuvent elles-même être synthétisées par la méthode décrite par WILLSTATTER et PUMMERER, Berichte, 37, pages 3734 à 3744, (1905), qui préconisent la décarboxylation de l'acide chélidonique lui-même obtenu, selon Organic Synthesis, Volume 2, page 41 et suivantes, par déshydratation du produit de la réaction de l'acétone sur l'oxalate d'éthyle.

A titre de simples illustrations non limitatives de l'invention, on donne les exemples suivants de réalisation du procédé selon l'invention.

Exemple 1

Synthèse de chlorure d'éthyl-2 hexanoyle.

Dans un réacteur de 250 ml équipé d'un agitateur mécanique, d'un thermomètre, d'un tube plongeant et d'un réfrigérant ascendant refroidi à —70°C, on a introduit une mole d'éthyl-2 hexanoïque et 10 millimole de N-méthyl pyridone-4. Le mélange a été porté alors à 115—120°C.

On a additionné du phosgène gazeux à cette température à raison de 120 g, en deux heures, en règlant le débit de phosgène de façon à observer un léger reflux au condenseur.

L'addition du phosgène étant achevée, on a continué d'agiter pendant 1 heure et demie, toujours à 115—120°C.

Après dégazage à l'azote, on a distillé sous pression réduite le chlorure d'éthyl-2 hexanoyle obtenu.

Le produit est d'une pureté satisfaisante. Le rendement est de 80% par rapport à l'acide, après distillation.

Exemple 2

O a utilisé le même mode opératoire que précédemment mais seulement 1 mM du même catalyseur. On a introduit 110 g de phosgène en 1 h 55. Le rendement obtenu est de 87%, après distillation.

Exemple 3

On a à nouveau abaissé la quantité de catalyseur utilisé. On a utilisé 0,1 mM de N-méthyl pyridone-4 et on a introduit 112 g de phosgène en 4 h 45', en respectant les autres conditions décrites aux exemples précédents.

On a obtenu un rendement de 89%, après distillation.

Exemple 4

Le mode opératoire de l'exemple 1 étant toujours adopté, on a utilisé 10 mM de N-butyl pyridone-4 et introduit 118 g de phosgène en 3 heures.

Le rendement en chlorure d'éthyl-2 hexanoyle a été de 86%, après distillation.

Exemple 5

On a utilisé cette fois-ci 10 mM de N-méthyl diméthyl-2,6 pyridone-4 et introduit 117 g de phosgène en 2 heures et demie. On a obtenu un rendement de 89% en chlorure d'éthyl-2 hexanoyle, après distillation.

Exemple 6

Dans un réacteur de 500 ml muni d'un agitateur, d'un thermomètre, d'un tube d'admission de gaz et d'un réfrigérant à reflux à —40°C on a placé 122 g d'acide benzoïque, 140,5 g de xylène et 0,96 g (0,01 mole) de 4H-pyranone-4.

On porte le milieu réactionnel à 120—125°C sous agitation et introduit le phosgène gazeux aussi rapidement que possible sans avoir de reflux notable au condenseur.

On introduit ainsi 112 g de phosgène en 2 h 30. On maintient la température encore 1 heure à 120°C dès la fin de l'introduction du phosgène après quoi on dégaze à l'azote pour achever d'éliminer gaz chlorhydrique, gaz carbonique et phosgène en excès.

On obtient ainsi un produit très pur (97%, d'après le taux de chlore hydrolysable) qui peut être purifié par distillation si on le souhaite. Le rendement est pratiquement de 100%.

Exemple 7

A titre de comparison avec l'exemple précédent, on a réitéré l'opération dans les mêmes conditions en remplaçant toutefois les 0,01 mole de 4H-$\gamma$-pyrone par 0,01 mole (2,84 g) de tétrabutylurée. La tétrabutylurée est un catalyseur du type carbonamide donnant assez peu de sous-produits et ceux-ci sont facilement séparables.

On constate alors que 3 h 30 sont nécessaires pour introduire tout le phosgène nécessaire (114 g), ce qui porte à 4 h 30 le temps total imposé pour atteindre la pureté rapportée à l'exemple 1.

Exemple 8

On a opéré avec le même appareillage qu'à l'exemple 6 et on a placé dans le réacteur 113 g (0,5 mole) d'anhydride benzoïque, 150, 5 g de xylène et 0,01 mole (0,96 g) de 4H-$\gamma$-pyrone. On porte le milieu obtenu à 100—115°C sous agitation et on introduit le phosgène comme indiqué à l'exemple 1.

Dans le tableau ci-après on a rapporté les quantités de phosgène introduites et le taux d'avancement de la réaction (déterminé par mesure du taux de chlore hydrolysable après dégazage soigneux de l'échantillon), en fonction du temps.

| Temps (mn) | Phosgène introduit | | Taux d'avancement (%) |
|---|---|---|---|
| | g | M | |
| 40 | 24,7 | 0,25 | 31 |
| 66 | 48,8 | 0,49 | |
| 80 | 56,2 | 0,57 | 91 |

On constate que la 4H-$\gamma$-pyrone permet une phosgénation rapide de l'anhydride benzoïque, au moins aussi rapide que pour l'acide du même nom.

Exemple 9

Si on opère strictement comme dans l'exemple 8, en remplaçant la 4H-$\gamma$-pyrone par de la tétrabutylurée (0,01 mole ou 2,84 g) on obtient le tableau suivant:

| Temps (mn) | Phosgène introduit | | Taux d'avancement (%) |
|---|---|---|---|
| | g | M | |
| 40 | 24,6 | 0,25 | 28 |
| 60 | 47,4 | 0,48 | |
| 80 | 54,2 | 0,55 | 60 |

On constate donc que la 4H-$\gamma$-pyrone permet une phosgénation beaucoup plus rapide qu'avec la tétrabutylurée.

Exemple 10

On a utilisé 0,01 mole (2,36 g) de dipropyl-2,6 diéthyl-3,5 pyranone-4, placé dans un réacteur de 250 ml, équipé comme à l'exemple 6, avec 144 g (1 mole) d'acide éthyl-2 hexanoïque.

On a porté le mélange à 100—115°C sous agitation et on a introduit 118 g de phosgène gazeux en 4 H 15 mn avec un très léger reflux au condenseur.

On maintient encore 1 H à 110°C sous agitation après quoi on élimine le phosgène en excès par dégazage à l'azote pour obtenir 157 g de chlorure d'acide brut, soit un rendement de 97%.

Après distillation sous pression réduite (104—106°C/90 mm Hg) effectuée dans un appareil équipé d'une simple colonne Vigreux, on recueille 146,3 g de chlorure d'éthyl-2 hexanoyle (rendement 90%) de pureté supérieure à 99%. Il reste 7 g de résidu dans le bouilleur..

On reproduit le mpele essai en utilisant comme catalyseur le résidu de 7 g sus-mentionné. On obtient ainsi 162,7 g de chlorure d'acide brut qui, après distillation, conduit à 129,2 g de produit de pureté supérieure à 99%, avec un résidu de distillation de 27 g.

Au bout de deux autres recyclages, la masse de résidu s'est stabilisée à 45 g environ.

Exemple 11

Synthèse de chlorure de benzoyle.

On a utilisé le mode opératoire décrit à l'exemple 1, appliqué à 1 mole d'acide benzoïque en solution dans 140 g de xylène. On a opéré en présence de 10 mM de N-butyl pyridone-4. On a introduit 120 g de phosgène en 2 h 40 mm. Le rendement obtenu après distillation est de 98,5%. Aucune trace d'anhydride benzoïque n'apparait en spectrométrie Infra-Rouge dans le résidu. Ce dernier a été recyclé et une nouvelle charge d'une mole d'acide benzoïque a été introduite dans le réacteur. On a introduit 120 g de phosgène en 3 heures et suivi le cycle habituel. On a obtenu un rendement de 96% en chlorure de benzoyle après distillation. Le résidu ne montrait toujours pas de traces d'anhydride en spectrométrie Infra-Rouge.

Exemple 12

On a opéré comme à l'exemple 6 avec 122 g d'acide benzoïque. 140,5 g de xylène et 0,01 mole (2,36 g) de dipropyl-2,6 diéthyl-3,5 pyranone-4.

On porte le mélange à 120°C sous agitation et introduit le phosgène aussi vite que possible sans toutefois avoir de reflux notable au condenseur. Après la fin de l'introduction du phosgène, on maintient le mélange réactionnel à 120°C pendant une heure après quoi on élimine le phosgène en excès par dégazage à l'azote.

Le chlorure de benzoyle obtenu a été séparé par distillation à l'aide d'une colonne Vigreux de pouvoir séparateur de l'ordre d'un plateau théorique. Le xylène part donc en tête entrainant une partie du chlorure d'acide. On a donc indiqué dans le tableau suivant seulement le rendement en chlorure d'acide distillé de pureté supérieure à 99.5%.

Pour montrer la possibilité de recycler plusieurs fois le catalyseur sans perte d'activité catalytique, on a effectué quatre recyclages successifs du résidu de distillation précédent toutes conditions égales par ailleurs. Les résultats obtenus sont rapportés dans le tableau ci-après.

| Recyclage n° | Durée de la phosgénation | Quantité de phosgène introduite | Rendement (1) | Résidu (catalyseur + anhydride) |
|---|---|---|---|---|
| 0 | 3 H | 111 g | 70% | 27 g |
| 1 | 3 H | 105 g | 71,4% | 34,5 g |
| 2 | 3 H 30 | 106 g | 75.5% | 46,2 g |
| 3 | 2 H 30 | 117 g | 73,3% | 46.6 g |
| 4 | 3 H | 110 g | 74% | 46,4 g |

(1) en produit distillé (pureté 99.5%).

On constate qu'il n'y a pas de perte de l'activité catalytique puisque la durée de la phosgénation est sensiblement constante, et que la teneur en anhydride se stabilise dès le deuxième recyclage, à une valeur propre au catalyseur et dépendant des constantes des vitesses de phosgénation de l'acide et de son anhydride.

Exemple 13

On utilise 0,96 g (0,01 mole) de 4H pyranone-4 et 88 g (1 mole) d'acide n-butyrique placés dans un réacteur de 250 ml équipé comme à l'exemple 6.

On porte le mélange à 100—115°C sous agitation et introduit le phosgène en 4 H environ de façon á avoir un léger reflux au condenseur.

Après la fin de l'introduction du phosgène, on maintient le mélange réactionnel à 95°C pendant 1 H, puis élimine le phosgène en excès par dégazage à l'azote anhydre.

Le chlorure de butyryle obtenu est séparé par distillation à l'aide d'une colonne Vigreux.

Pour montrer ici encore la possibilité de recycler plusieurs fois le catalyseur sans perte notable d'activité catalytique, on a effectué 2 recyclages successifs du résidu de distillation précedent, toutes conditions égales par ailleurs.

Les résultats obtenus sont consignés dans le tableau ci-après qui indique également le résultat d'un essai effectué dans les mêmes conditions mais sans catalyseur.

| Essai | Catalyseur | Quantité de phosgène introduite | Rendement (1) | Quantité de résidu |
|---|---|---|---|---|
| comparaison | néant | 100 g | 0 | — |
| 1 | 4 H pyranone-4 0,01 mole | 108 g | 75% | 11,1 g |
| 2 | résidu de l'essai 1 | 108 g | 76% | 5,2 g |
| 3 | résidu de l' essai 2 | 108 g | 75% | 11 g |

(1) en produit distillé (pureté > 99%)

Exemple 14

On a utilisé 4 kg d'acide éthyl-2 hexanoïque soit 38 moles et 34,5 g de 2-6 diméthyl-$\gamma$-pyranone, soit 0,277 mole placés dans un réacteur de 10 litres.

On a porté le mélange à 90°C et envoyé le phosgène de façon à avoir un léger reflux sur les condenseurs du réacteur; on a introduit ainsi 4 kg de phosgène, soit 40,6 moles, en 2 H 45 mn. Après une heure d'agitation on dégaze le contenu du réacteur par envoi d'azote de façon à chasser l'acide chlorhydrique, le gaz carbonique et le phosgène en excès; par distillation sous pression réduite on obtient 4, 330 kg de chlorure d'acide titrant 98% soit un rendement de 96%.

Exemple 15

Dans un réacteur de 10 litres, on introduit 3, 905 kg d'acide éthyl-2 hexanoïque (36,9 moles) et 26 g de 4 H-$\gamma$-pyranone (0,26 mole).

On porte le mélange à 130°C sous agitation et on introduit le phosgène gazeux à raison de 8,33 mole/H de façon à maintenir un très léger reflux sur le condenseur de réacteur.

On introduit ainsi 3,000 kg de phosgène en 4 heures. On laisse sous agitation 1 heure à 130°C après quoi on dégaze à l'azote le produit obtenu pour éliminer l'acide chlorhydrique, le gaz carbonique et le phosgène en excès.

On distille le chlorure d'acide éthyl-2 hexanoïque sous vide, $Eb_{10} = 62°C$, on obtient ainsi 4,285 kg de chlorure d'acide à 99%, soit un rendement de 97,4% et 130 g de queues de distillation constituées par la charge catalytique et un peu de chlorure d'acide résiduel.

Ces queues de distillation sont recyclées avec une charge de 3,905 kg d'acide éthyl-2 hexanoïque frais portées à 130°C et phosgénées dans les conditions précédentes, le débit de phosgène permettant d'obtenir un reflux très léger sur le condenseur est de 9,4 moles/H après 3 H 40 de phosgénation on obtient un reflux important sur le condenseur indiquant la fin de réaction. On laisse réagir 3 heures, on dégaze, puis distille dans les conditions précédentes et on obtient 3,9 kg de chlorure d'acide et 0,7 kg de queues qui sont recyclées dans une opération suivante.

La tableau suivant donne les durées de réaction, quantités de queues de distillation et rendement en chlorure d'acide obtenus dans une série d'opérations de recyclage.

| Opération | Durée de réaction totale | Quantité de queues de distillation recyclées | Rendement en chlorure d'acide |
|---|---|---|---|
| 1 | 4 heures | 0,130 kg | 97,4% |
| 2 (1er recyclage) | 7 heures | 0,700 kg | 85% |
| 3 (3ème recyclage) | 7 heures | 0,700 kg | 83,4% |
| 4 (3ème recyclage) | 7 heures | 0,700 kg | 88,9% |

Exemple 16

Synthèse de chlorure d'acryloyle

On a utilisé le même mode opératoire qu'à l'exemple 1, appliqué à 1 mole d'acide acrylique, en présence de 10 mM de N-butyl pyridone-4.

On a introduit 113 g de phosgène en 3 h 10 mn.

On a obtenu du chlorure d'acryloyle avec un rendement de 64% après distillation.

**Revendications**

1. Procédé de fabrication de chlorures d'acides organiques par phosgénation à chaud des acides ou des anhydrides d'acides correspondants en présence d'un catalyseur, caractérisé en ce qu'on utilise comme catalyseur au moins un membre de la famille de formule générale (I)

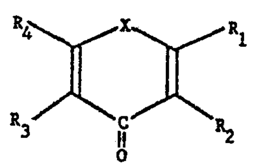

où X est un atome d'oxygène ou un atome d'azote monosubstitué par un groupe $R_5$ qui est un groupe alkyle comprenant de 1 à 6 atomes de carbone, et où $R_1$, $R_2$, $R_3$ $R_4$ sont tous différentes ou semblables pour au moins deux d'entre eux et sont un atome d'hydrogène ou un radical organique susceptible ou non de réagir avec le phosgène et contenant au plus 20 atomes de carbone.

2. Procédé de fabrication selon la revendication 1, caractérisé en ce que la proportion de catalyseur par rapport à l'acide ou à l'anhydride est comprise entre 0,01 et 10% en poids, de préférence entre 0,1 et 2% en poids.

3. Procédé de fabrication selon l'une des revendications 1 ou 2, caractérisé en ce que le catalyseur est un membre de la famille de formule générale

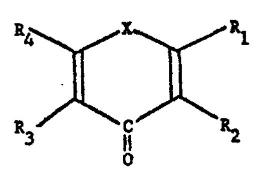

dans laquelle d'une part X = O ou N—$N_5$, $R_5$ étant un groupe alkyl linéaire ou ramifié de $C_1$ à $C_6$, et, d'autre part: —$R_1$ = H, alkyl linéaire ou ramifié de $C_1$ à $C_{12}$, substitué ou non par des atomes de chlore, des hydroxyles, des carboxyles, des groupes esters ou des groupes éthers, aryle substitué ou non par un ou des groupes halogène, hydroxyle ou carboxyle, aralkyle de $C_7$ à $C_{20}$, alkaryle de $C_7$ à $C_{20}$, hydroxyle, carboxyle, dialkylamino de $C_1$ à $C_3$,

$R_2$ et $R_3$ sont identiques ou différents et ont l'une des significations possibles pour $R_1$ et, éventuellement, la même signification que $R_1$, ou bien lorsque X = N—$R_5$, sont de préférence l'un et l'autre un atome d'hydrogène.

$R_4$ a l'une des significations possibles pour $R_1$, et éventuellement a la même signification que $R_1$, et/ou que $R_2$, et/ou $R_3$, ou bien dans laquelle $R_1$ et $R_2$ pris ensemble et/ou $R_3$ et $R_4$ pris ensemble forment un groupe phényle.

4. Procédé de fabrication selon la revendication 3, caractérisé en ce que le catalyseur est un membre de la famille de formule générale:

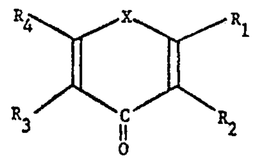

dans laquelle d'une part $X = O$ ou $N—R_5$, $R_5$ étant un groupe alkyl linéaire ou ramifié de $C_1$ à $C_6$, et, d'autre part, $R_1 = R_2 = R_3 = R_4 = H$ ou bien dans laquelle:

$R_1 = R_4 =$ alkyl de $C_1$ à $C_6$, $R_2 = R_3 = H$

ou bien dans laquelle $X = O$ et:

$R_1 = R_4 =$ alkyl de $C_1$ à $C_6$ et $R_2$, $R_3 =$ alkyl de $C_1$ à $C_6$

$R_1 = R_4 = H$ et $R_2$, $R_3 =$ aryle substitué ou non par des groupes halogène, hydroxyle ou carboxyle,

$R_1 = R_4 = H$ et $R_2$, $R_3 =$ aralkyle de $C_7$ à $C_{20}$ ou alkaryle de $C_7$ à $C_{20}$

$R_1 = R_4 = COOH$ et $R_2$, $R_3 = H$ ou alkyle de $C_1$ à $C_6$

5. Procédé de fabrication selon la revendication 4, caractérisé en ce que le catalyseur est la 4H-$\gamma$-pyranone-4.

6. Procédé de fabrication selon la revendication 4, caractérisé en ce que le catalyseur est la N-butyl pyridone-4.

7. Procédé selon l'une quelconque des revendications précédentes, caractérisé en ce qu'on effectue la réaction en utilisant comme solvant le chlorure d'acide qu'on cherche à préparer.

**Patentansprüche**

1. Verfahren zur Herstellung von Carbonsäurechloriden durch Phosgenierung der entsprechenden Carbonsäuren oder ihrer Anhydride unter Erwärmen in Gegenwart eines Katalysators, dadurch gekennzeichnet daß als Katalysator mindestens eine Verbindung der allgemeinen Formel I

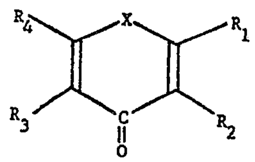

verwendet wird, in der bedeuten: X ein Sauerstoffatom oder ein mit einer Gruppe $R_5$ monosubstituiertes Stickstoffatom, die eine $C_1$- bis $C_6$-Alkylgruppe darstellt, $R_1$, $R_2$, $R_3$ und $R_4$ ein Wasserstoffatom oder eine organische Gruppe mit höchstens 20 C-Atomen, die gegebenenfalls mit Phosgen reaktiv ist, wobei die Substituenten $R_1$ bis $R_4$ sämtlich unterschiedlich oder mindestens zwei von ihnen gleich sind.

2. Verfahren nach Anspruch 1, dadurch gekennzeichnet, daß die Menge des Katalysators 0,01 bis 10 Gew.-% und vorzugsweise 0,1 bis 2 Gew.-%, bezogen auf die Carbonsäure oder ihr Anhydrid, beträgt.

3. Verfahren nach Anspruch 1 oder 2, dadurch gekennzeichnet, daß als Katalysator eine Verbindung der allgemeinen Formel

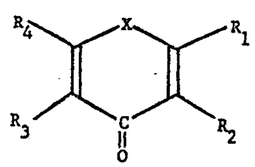

verwendet wird, in der bedeuten: $X = O$ oder $N—R_5$, wobei $R_5$ ein geradkettiges oder verzweigtes $C_1$- bis $C_6$-Alkyl darstellt, $R_1$ H, geradkettiges oder verzweigtes und gegebenenfalls mit Chloratomen, Hydroxygruppen, Carboxylgruppen, Estergruppen oder Äthergruppen substituiertes $C_1$- bis $C_{12}$-Alkyl, gegegebenenfalls mit Halogenatomen, Hydroxylgruppen oder Carboxylgruppen substituiertes Aryl, $C_7$- bis $C_{20}$-Aralkyl, $C_7$- bis $C_{20}$-Alkaryl, Hydroxyl, Carboxyl oder $C_1$- bis $C_3$-Dialkylamino, $R_2$ und $R_3$ zugleich oder unabhängig eine der Bedeutungen von $R_1$ und gegebenenfalls das gleiche wie $R_1$ und vorzugsweise, wenn $X = N—R_5$ ist, zugleich Wasserstoff und $R_4$ eine der Bedeutungen von $R_1$ und gegebenenfalls die gleiche Bedeutung wie $R_1$ und/oder wie $R_2$ und/oder wie $R_3$, wobei $R_1$ und $R_2$ und/oder $R_3$ und $R_4$ auch zusammen eine Phenylgruppe bilden können.

4. Verfahren nach Anspruch 3, dadurch gekennzeichnet, daß als Katalysator eine Verbindung der allgemeinen Formel

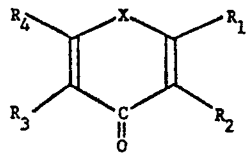

verwendet wird, in der bedeuten:
X O oder N—$R_5$, wobei $R_5$ geradkettiges oder verzweigtes $C_1$- bis $C_6$-Alkyl darstellt,
und
$R_1 = R_2 = R_3 = R_4 = H$
oder
$R_1 = R_4 = C_1$- bis $C_6$-Alkyl und
$R_2 = R_3 = H$
oder
$X = O$ und
$R_1 = R_4 = C_1$- bis $C_6$-Alkyl und
$R_{21}, R_3 = C_1$- bis $C_6$-Alkyl oder
$R_1 = R_4 = H$ und
$R_2, R_3 =$ gegebenenfalls mit Halogenatomen, Hydroxylgruppen oder Carboxylgruppen substituiertes Aryl oder
$R_1 = R_4 = H$ und
$R_2, R_3 = C_7$- bis $C_{20}$-Aralkyl oder $C_7$- bis $C_{20}$-Alkaryl oder
$R_1 = R_4 = COOH$ und
$R_2, R_2 = H$ oder $C_1$- bis $C_6$-Alkyl.

5. Verfahren nach ˙anspruch 4, dadurch gekennzeichnet, daß als Katalysator 4H-$\gamma$-Pyranon-4 verwendet wird.

6. Verfahren nach Anspruch 4, dadurch gekennzeichnet, daß als Katalysator N-Butylpyridon-4 verwendet wird.

7. Verfahren nach einem der vorhergehenden Ansprüche, dadurch gekennzeichnet, daß die Reaktion unter Verwendung des herzustellenden Säurechlorids als Lösungsmittel durchgeführt wird.

**Claims**

1. Process for the manufacture of organic acid chlorides by the phosgenation, under the action of heat, of the corresponding acids or acid anhydrides in the presence of a catalyst, characterised in that at least one member of the family of the general formula (I)

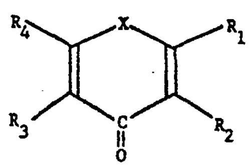

is used as the catalyst, in which formula X is an oxygen atom or a nitrogen atom which is monosubstituted by a group $R_5$, which is an alkyl group containing from 1 to 6 carbon atoms, and in which formula $R_1$, $R_2$, $R_3$ and $R_4$ are all different, or at least two or them are the same, and are a hydrogen atom or an organic radical which may or may not be capable of reacting with phosgene and contains at most 20 carbon atoms.

2. Process of manufacture according to Claim 1, characterised in that the proportion of catalyst, relative to the acid or to the anhydride, is between 0.01 and 10% by weight and preferably between 0.1 and 2% by weight.

3. Process of manufacture according to one of Claims 1 or 2, characterised in that the catalyst is a member of the family of the general formula

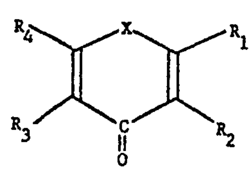

in which, on the one hand, $X = O$ or N—$R_5$ being a linear or branched $C_1$ to $C_6$ alkyl group, and, on the other hand: $R_1 = H$, a linear or branched $C_1$ to $C_{12}$ alkyl group which may or may not be substituted by chlorine atoms, hydroxyl groups, carboxyl groups, ester groups or ether groups, an aryl group which

may or may not be substituted by one or more halogen, hydroxyl or carboxyl groups, a $C_7$ to $C_{20}$ aralkyl group, a $C_7$ to $C_{20}$ alkaryl group, a hydroxyl group, a carboxyl group, or a $C_1$ to $C_3$ dialkylamino group, $R_2$ and $R_3$ are identical or different and have one of the possible meanings for $R_1$, and optionally the same meaning as $R_1$, or, if $X = N—R_5$, they are both preferably a hydrogen atom, and $R_4$ has one of the possible meanings for $R_1$, and optionally has the same meaning as $R_1$ and/or as $R_2$ and/or $R_3$, or alternatively $R_1$ and $R_2$ taken together, and/or $R_3$ and $R_4$ taken together, form a phenyl group.

4. Process of manufacture according to Claim 3, characterised in that the catalyst is a member of the family of the general formula:

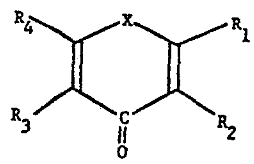

in which, on the one hand, X = O or $N—R_5$, $R_5$ being a linear or branched $C_1$ to $C_6$ alkyl group and, on the other hand:

$R_1 = R_2 = R_3 = R_4 = H$,

or alternatively:

$R_1 = R_4 = C_1$ to $C_6$ alkyl and $R_2 = R_3 = H$,

or alternatively in which X = O and:

$R_1 = R_4 = C_1$ to $C_6$ alkyl and $R_2$ and $R_3 = C_1$ to $C_6$ alkyl,

$R_1 = R_4 = H$ and $R_2$ and $R_3$ = aryl which may or may not be substituted by halogen, ydroxyl or carboxyl groups,

$R_1 = R_4 = H$ and $R_2$ and $R_3 = C_7$ to $C_{20}$ aralkyl or $C_7$ to $C_{20}$ alkaryl, or

$R_1 = R_4 = COOH$ and $R_2$ and $R_3 = H$ or $C_1$ or $C_6$ alkyl.

5. Process of manufacture according to Claim 4, characterised in that the catalyst is 4H-pyran-4-one.

6. Process of manufacture according to Claim 4, characterised in that the catalyst is N-butyl-4-pyridone.

7. Process according to any one of the preceding claims, characterised in that the reaction is carried out using, as the solvent, the acid chloride which it is desired to prepare.